# EUROPÄISCHE PATENTSCHRIFT

(11) **EP 2 793 777 B1**
(45) Veröffentlichungstag und Bekanntmachung des Hinweises auf die Patenterteilung: **09.12.2015**
(21) Anmeldenummer: 12816275.7
(22) Anmeldetag: 20.12.2012
(51) Int. Cl.: A61F 13/26, A61F 13/20

(54) **VERFAHREN ZUR MONTAGE VON APPLIKATORTAMPONS**
METHOD FOR ASSEMBLING APPLICATOR TAMPONS
PROCÉDÉ DE MONTAGE DE TAMPONS APPLICATEURS

(30) Priorität: 23.12.2011 DE 102011056969
(43) Veröffentlichungstag der Anmeldung: 29.10.2014
(73) Patentinhaber: Kröning, Thomas, 01239 Dresden (DE)
(72) Erfinder: Kröning, Thomas, 01239 Dresden (DE)
(74) Vertreter: Bauer, Steffen
(86) Internationale Anmeldenummer: PCT/EP2012/076418
(87) Internationale Veröffentlichungsnummer: WO 2013/092880

(56) Entgegenhaltungen:
- EP-A1- 2 335 666
- GB-A- 594 580

## Beschreibung

Die Erfindung betrifft ein Verfahren zur Montage von Applikatortampons, die aus einem Tamponstopfen mit Rückholfaden sowie einem Außen- und einem Innenröhrchen bestehen.

Wesentliche Merkmale sind eine glatte Oberfläche sowie ein stabiler und dennoch leicht öffnender vorderer Bereich des Außenröhrchens, hohe Saugfähigkeit und Verträglichkeit des Tamponstopfens und bequeme Handhabbarkeit. Zunehmend achtet der Markt auch auf die Umweltverträglichkeit der Produkte, insbesondere auf umweltverträgliche Entsorgungsfähigkeit sowohl der Applikatoren als auch des Tamponstopfens mitsamt dem Rückholfaden. Bevorzugt kommen deshalb biologisch abbaubare Materialien zum Einsatz.

Bei der Herstellung von Applikatortampons müssen sowohl leicht verformbare Bestandteile, nämlich Tamponstopfen und Rückholfaden, als auch formstabile Bestandteile, nämlich die Applikatorröhrchen, gehandhabt und miteinander verbunden werden. Dabei stellen einerseits der Schutz des Kopfes des Tamponstopfens und andererseits der Einzug des Rückholfadens in das Applikatorinnenröhrchen die größten Herausforderungen dar. Eine bekannte Lösung diesbezüglich beschreibt die GB 594,580 in Form eines Verfahrens und einer Vorrichtung zur Montage von Tampons in Applikatoren von Menstruationstampons. Die bekannte Lösung umfasst eine Führungsschiene, in welche der Tamponstopfen mit Rückholfaden und die Applikatorröhrchen platziert werden. Die Führungsschiene ist durch eine Fuge unterteilt, die mit einer darunter angeordneten Mulde verbunden ist. Vor der Montage sind die Applikatorröhrchen hintereinander und axial ausgerichtet auf einer Seite der Fuge und der Tamponstopfen auf der anderen Seite der Fuge angeordnet, so dass der Rückholfaden in der Mulde abgelegt ist. Die Führungsschiene weist zur achsgleichen Aufnahme der Applikatorröhrchen einen Durchmesserabsatz auf, an den das Außenröhrchen stößt. Die Führungsschiene ist auf der für die Aufnahme der Applikatorrörchen vorgesehenen Seite neben der Fuge trichterförmig ausgebildet. Nach dem Beladen der Führungsschiene erfolgt die Montage derart, dass mittels eines röhrenförmigen Druckstempels das Innen- in das Außenröhrchen gedrückt, mittels am offenen Ende des Druckstempels anliegenden Unterdrucks der Tamponrückholfaden in das Innenröhrchen gesaugt und mittels eines zweiten Druckstempels der Tamponstopfen durch den Trichter stirnseitig in das von dem Schienenabsatz gehaltene Außenröhrchen gedrückt wird. Die Montageschritte können gleichzeitig ausgeführt werden. Allerdings zeigt sich bei der bekannten Lösung das Problem, dass ein glatter Verschluss der Stirnseite des Außenröhrchens nur sehr aufwendig möglich ist, da der Tamponstopfen durch die Stirnseite in das Außenröhrchen gelangt und deshalb der Verschluss erst nachträglich erfolgen kann, etwa durch Kopfformung. Diese Art des Verschließens des Außenröhrchens ist zudem bei aus biologisch abbaubaren Kunststoffen bestehenden Applikatoren wegen der Unmöglichkeit der nachträglichen Verformung ausgeschlossen.

Daraus ergibt sich als Aufgabe der Erfindung, die Herstellung von Applikatortampons zu vereinfachen und insbesondere den nachträglichen Verschluss des Applikators zu vermeiden.

Die Aufgabe wird erfindungsgemäß durch ein Verfahren nach Patentanspruch 1 gelöst. Das erfindungsgemäße Verfahren bedient sich eines röhrenförmigen Montagekanals mit einem ersten Abschnitt mit einem ersten Durchmesser und einem zweiten Abschnitt mit einem zweiten, gegenüber dem ersten kleineren Durchmesser. Die beiden Abschnitte stoßen axial ausgerichtet aneinander und bilden derart einen Absatz. Die distale Öffnung des zweiten Abschnitts erweitert sich trichterförmig. Nahe dem Absatz mündet ein Seitenkanal in den zweiten Abschnitt. Ein Druckluftsystem ist mit der distalen Öffnung des zweiten Abschnitts und dem Seitenkanal distal verbindbar. Mittels eines Druckstempels werden ein Tampon mit Tamponrückholfaden und ein Applikatorinnenröhrchen in ein Applikatoraußenröhrchen gedrückt, wobei
- das Applikatoraußenröhrchen im ersten Abschnitt des Montagekanals so positioniert wird, dass die Öffnung des Applikatoraußenröhrchens am Absatz anliegt,
- der Tampon durch den zweiten Abschnitt des Montagekanals in das Applikatoraußenröhrchen so eingeschoben wird, dass der Tamponrückholfaden im zweiten Abschnitt des Montagekanals verbleibt,
- durch die distale Öffnung des zweiten Abschnitts Druckluft in den Montagekanal eingeblasen wird, so dass der Tamponrückholfaden in den Seitenkanal erstreckt wird,
- das Applikatorinnenröhrchen im zweiten Abschnitt des Montagekanals so positioniert wird, dass die Mündung des Seitenkanals frei bleibt,
- Druckluft durch den Seitenkanal in den Montagekanal eingeblasen wird, so dass der Tamponrückholfaden in das Applikatorinnenröhrchen erstreckt wird,
- das Applikatorinnenröhrchen bis zum Erreichen seiner finalen Position in das Applikatoraußenröhrchen geschoben wird und abschließend
- der Applikatortampon aus dem Montagekanal ausgestoßen wird.

Eine vorteilhafte Ausgestaltung der Erfindung besteht darin, dass der Ausstoß des Applikatortampons aus dem Montagekanal mittels Druckluft erfolgt, die durch die distale Öffnung des zweiten Abschnitts in den Montagekanal eintritt. Weitergeführt wird dieser erfindungsgemäße Gedanke, indem der Ausstoß des Applikatortampons mittels Druckluft erfolgt, die durch den Seitenkanal oder sowohl durch die distale Öffnung des zweiten Abschnitts als auch durch den Seitenkanal in den Montagekanal eintritt.

Durch die Erfindung können Applikatoraußenröhrchen mit vorgeformter Stirnseite verwendet werden. Dazu gehören auch solche, die einstückig sind und/oder aus einem biologisch abbaubaren Kunststoff bestehen.

Die Erfindung wird im Folgenden in Form eines bevorzugten Ausführungsbeispiels anhand der Zeichnung näher erläutert. Die Zeichnung zeigt

die einzelnen Verfahrensschritte anhand schematischer Schnittfiguren 1 bis 8.

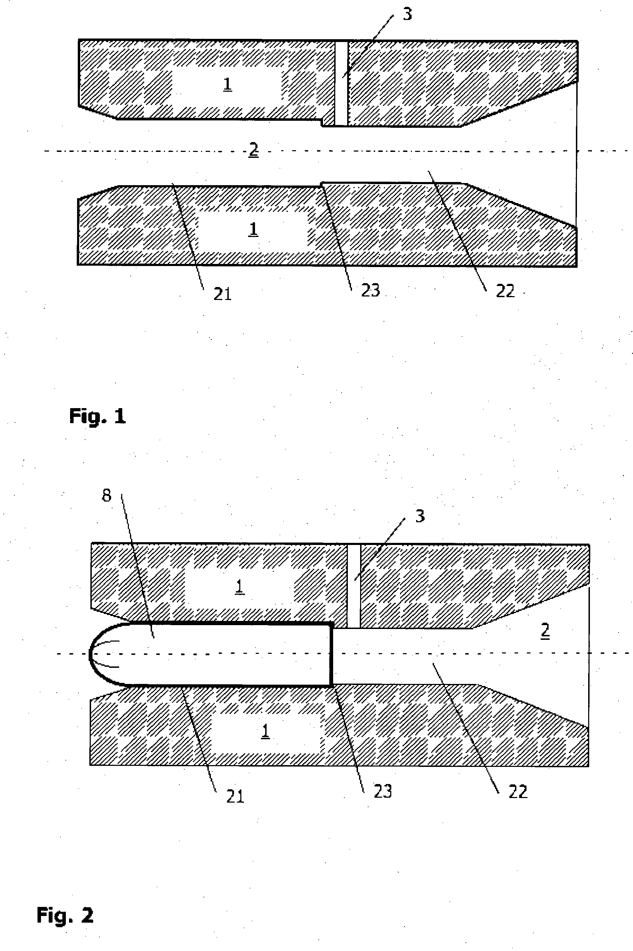

In Fig. 1 ist ein zur Herstellung von Applikatortampons geeigneter Montagekanal 2 dargestellt. Der Montagekanal 2 befindet sich in einer Montageanordnung 1 und umfasst einen ersten Abschnitt 21 und einen zweiten Abschnitt 22. Die Abschnitte 21 und 22 sind axial ausgerichtet, wobei der Durchmesser des zweiten Abschnitts 22 kleiner ist als der Durchmesser des ersten Abschnitts 21, so dass ihr Übergang durch einen den Durchmessersprung charakterisierenden Absatz 23 gebildet wird. Der Durchmesser des ersten Abschnitts 21 ist abgestimmt auf den Außendurchmesser eines Applikatoraußenröhrchens während der Durchmesser des zweiten Abschnitts 22 geringfügig über dem Innendurchmesser eines Applikatoraußenröhrchens liegt. Die Öffnungen des ersten und des zweiten Abschnitts erweitern sich trichterförmig oder konisch. Nahe dem Absatz 23 mündet ein Seitenkanal 3 in den Abschnitt 22, dessen Durchmesser auf die Stärke eines Tamponrückholfadens abgestimmt ist.

Fig. 2 zeigt den ersten Verfahrensschritt zur Herstellung von Applikatortampons. Ein Applikatoraußenröhrchen 8 wird im ersten Abschnitt 21 des Montagekanals 2 angeordnet, wobei die Stirnseite des Applikatoraußenröhrchens 8 zur Öffnung des Montagekanals 2 zeigt und die Öffnung des Applikatoraußenröhrchens 8 am Absatz 23 anliegt.

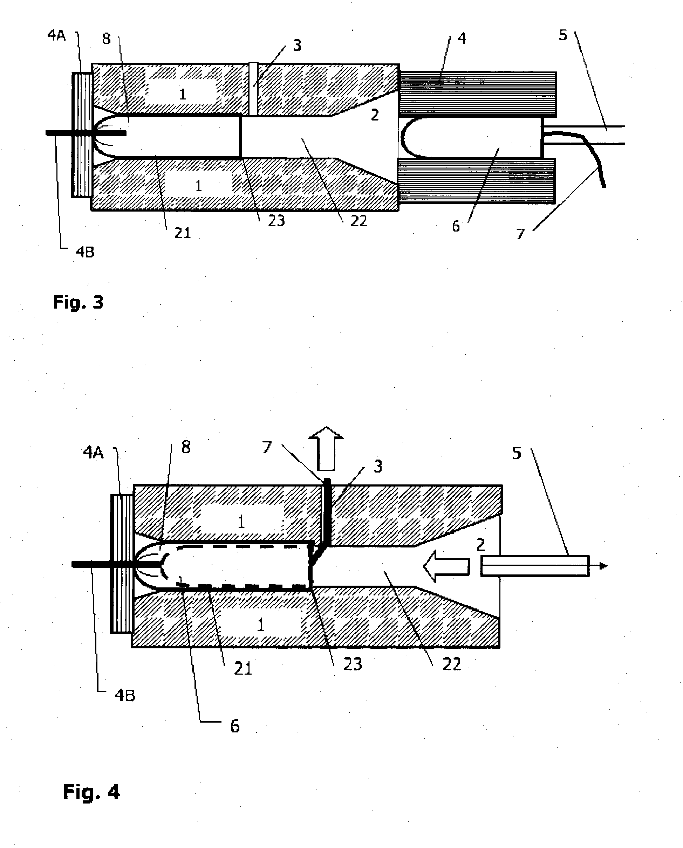

Fig. 3 zeigt den zweiten Verfahrensschritt. An die Montageanordnung 1 ist eine Zuführeinrichtung 4 derart angedockt, dass mittels eines Einschubfingers 5 ein Tamponstopfen 6 mit einem Rückholfaden 7 durch die trichterförmige Erweiterung der Öffnung des zweiten Abschnitts 22 in den Montagekanal 2 und das Applikatoraußenröhrchen 8 eingeschoben wird. Die Öffnung des Abschnitts 21 ist mittels einer Deckplatte 4A verschlossen, indem die Montageeinrichtung 1 vorteilhaft ausgeführt als Rundtakt- oder Revolvervorrichtung entsprechend mit einer feststehenden Deckplatte 4A ausgestattet ist, in deren Bereich der zweite Verfahrensschritt und die folgenden Verfahrensschritte ausgeführt werden. Die Montageeinrichtung verfügt ferner über einen die Deckplatte 4A durchdringenden und axial verschiebbaren Haltestift 4B, der als Anschlag für den Tamponstopfen 6 dient, wie aus Fig. 4 deutlich wird.

Fig. 4 zeigt den dritten Verfahrensschritt. Die trichterförmige Öffnung des zweiten Abschnitts 22 des Montagekanals 2 wird mit einer Druckluftquelle 10 verbunden. Mittels in den Montagekanal 2 eingeblasener Druckluft wird der Tamponrückholfaden 7 in den Seitenkanal 3 verlegt, durch den die Druckluft entweicht. Der Einschubfinger 5 wird zurückgezogen.

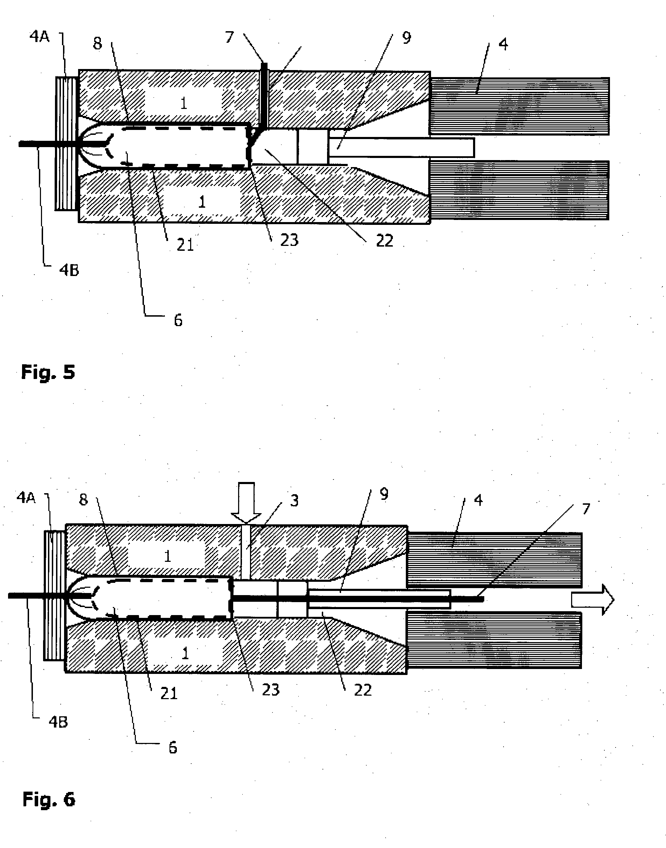

Fig. 5 zeigt den vierten Verfahrensschritt. Die Zuführeinrichtung 4 ist an die Montageanordnung 1 angedockt, so dass der Einschubfinger 5 ein Applikatorinnenröhrchen 9 durch die trichterförmige Öffnung in den zweiten Abschnitt 22 des Montagekanals 2 befördert. Das Applikatorinnenröhrchen 9 wird derart positioniert, dass sich der vorderste Rand kurz vor der Mündung des Seitenkanals 3 befindet, der Seitenkanal 3 also mit dem zweiten Abschnitt 22 des Montagekanals 2 verbunden bleibt.

Fig. 6 zeigt den fünften Verfahrensschritt. Der Seitenkanal 3 wird distal an die Druckluftquelle 10 angeschlossen, so dass mittels Druckluft der Tamponrückholfaden 7 in das Applikatorinnenröhrchen 9 befördert wird, da die Druckluft ausschließlich durch das Applikatorinnenröhrchen 9 aus dem Montagekanal 2 entweichen kann.

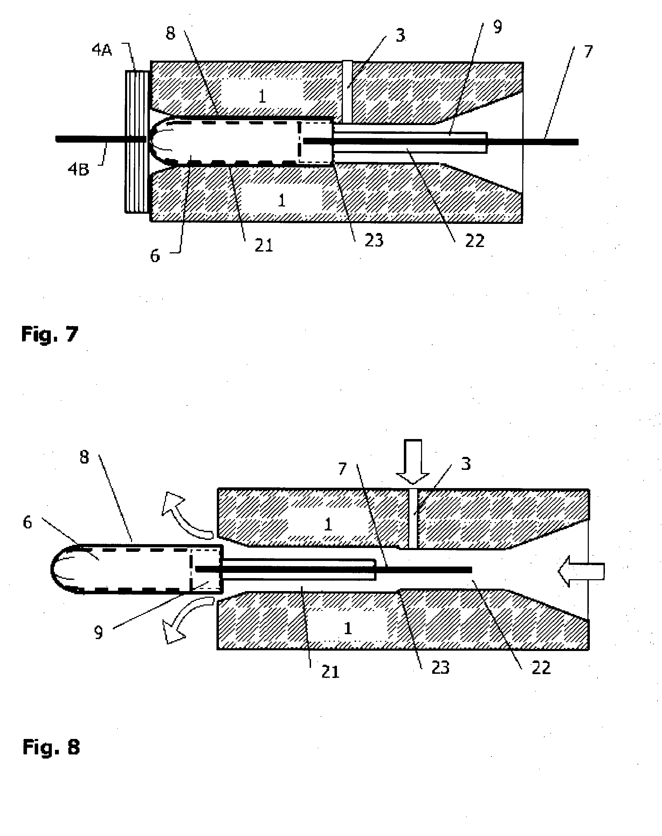

Fig. 7 zeigt den sechsten Verfahrensschritt. Das Applikatorinnenröhrchen 9 wird in das Applikatoraußenröhrchen 8 gedrückt, so dass es seine finale Position erreicht. Dabei wird der Haltestift 4B zurückgefahren und der Tamponstopfen 6 durch den Vorschub des Applikatorinnenröhrchens 9 ebenfalls in die finale Position befördert.

Fig. 8 zeigt den siebten Verfahrensschritt. Der erste Abschnitt 21 des Montagekanals 2 wird geöffnet und der montierte Applikatortampon wird aus dem Montagekanal 2 ausgestoßen. Der Ausstoß erfolgt durch Druckluft, die über den zweiten Abschnitt 22 und/oder den Seitenkanal 3 in den Montagekanal 2 eingeleitet wird.

Die vorstehend im Einzelnen aufgeführten Verfahrensschritte können selbstverständlich in geeigneter Weise zeitlich miteinander kombiniert ausgeführt werden, um eine möglichst kurze Zykluszeit zu realisieren. Die Montageanordnung 1 und die Zuführeinrichtung 4 sind dem Fachmann alls Bestandteile von Montageautomaten hinlänglich geläufig und bedürfen keiner weiteren Erläuterung. Gleiches gilt in Bezug auf die Bereitstellung der Druckluft und die Auswahl geeigneter Anschlüsse. Für die Ausgestaltung der Steuerung einschließlich einer programmtechnischen Umsetzung des erfindungsgemäßen Verfahrens in die Programmierung der Steuerung stehen dem Fachmann ebenfalls bekannte Werkzeuge zur Verfügung.

## Patentansprüche

1. Verfahren zur Herstellung von Applikatortampons unter Verwendung eines röhrenförmigen Montagekanals (2) mit einem ersten Abschnitt (21) mit einem ersten Durchmesser und einem zweiten Abschnitt (22) mit einem zweiten, gegenüber dem ersten kleineren Durchmesser, wobei die beiden Abschnitte axial ausgerichtet aneinander stoßen und derart einen Absatz (23) bilden, die distale Öffnung des zweiten Abschnitts (22) sich trichterförmig erweitert und nahe dem Absatz (23) ein Seitenkanal (3) in den zweiten Abschnitt (22) mündet, sowie eines Druckluftsystems, das mit der distalen Öffnung des zweiten Abschnitts (22) und dem Seitenkanal (3) distal verbindbar ist, indem mittels eines Druckstempels (5) ein Tampon (6) mit Tamponrückholfaden (7) und ein Applikatorinnenröhrchen (9) in ein Applikatoraußenröhrchen (8) gedrückt werden, **dadurch gekennzeichnet, dass** das Applikatoraußenröhrchen (8) im ersten Abschnitt (21) des Montagekanals (2) so positioniert wird, dass die Öffnung des Applikatoraußenröhrchens (8) am Absatz (23) anliegt, der Tampon (6) durch den zweiten Abschnitt (22) des Montagekanals (2) in das Applikatoraußenröhrchen (8) so eingeschoben wird, dass der Tamponrückholfaden (7) im zweiten Abschnitt (22) des Montagekanals (2) verbleibt, durch die distale Öffnung des zweiten Abschnitts (22) Druckluft in den Montagekanal (2) eingeblasen wird, so dass der Tamponrückholfaden (7) in den Seitenkanal (3) erstreckt wird, das Applikatorinnenröhrchen (9) im zweiten Abschnitt (22) des Montagekanals (2) so positioniert wird, dass die Mündung des Seitenkanals (3) frei bleibt, Druckluft durch den Seitenkanal (3) in den Montagekanal (2) eingeblasen wird, so dass der Tamponrückholfaden (7) in das Applikatorinnenröhrchen (9) erstreckt wird, das Applikatorinnenröhrchen (9) bis zum Erreichen seiner finalen Position in das Applikatoraußenröhrchen (8) geschoben wird und abschließend der Applikatortampon (6, 7, 8, 9) aus dem Montagekanal (2) ausgestoßen wird.

2. Verfahren nach Anspruch 1, **dadurch gekennzeichnet, dass** der Ausstoß des Applikatortampons (6, 7, 8, 9) aus dem Montagekanal (2) mittels Druckluft erfolgt, die durch die distale Öffnung des zweiten Abschnitts (22) in den Montagekanal (2) eintritt.

3. Verfahren nach Anspruch 1 oder 2, **dadurch gekennzeichnet, dass** der Ausstoß des Applikatortampons (6, 7, 8, 9) mittels Druckluft erfolgt, die durch den Seitenkanal (3) in den Montagekanal (2) eintritt.

## Claims

1. A method for producing applicator tampons using a tubular assembly channel (2) with a first section (21) having a first diameter and a second section (22) having a second diameter smaller than the first one, wherein the two sections adjoin each other in axial orientation and thereby form a shoulder (23), the distal opening of the second section (22) widens in a funnel shape and, near the shoulder (23), a side channel (3) opens into the second section (22), and using a compressed air system which can be connected distally to the distal opening of the second section (22) and to the side channel (3), in which a tampon (6) with tampon retrieval thread (7) and an applicator inner tube (9) is pressed into an applicator outer tube (8) by means of a plunger (5), **characterized in that** the applicator outer tube (8) is positioned in the first section (21) of the assembly channel (2) in such a way that the opening of the applicator outer tube (8) bears on the shoulder (23), the tampon (6) is pushed through the second section (22) of the assembly channel (2) into the applicator outer tube (8) in such a way that the tampon retrieval thread (7) remains in the second section (22) of the assembly channel (2), compressed air is blown into the assembly channel (2) through the distal opening of the second section (22), in such a way that the tampon retrieval thread (7) is extended into the side channel (3), the applicator inner tube (9) is positioned in the second section (22) of the assembly channel (2) in such a way that the mouth of the side channel (3) remains free, compressed air is blown through the side channel (3) into the assembly channel (2), such that the tampon retrieval thread (7) is extended into the applicator inner tube (9), the applicator inner tube (9) is pushed into the applicator outer tube (8) until it reaches its final position, and, finally, the applicator tampon (6, 7, 8, 9) is ejected from the assembly channel (2).

2. The method as claimed in claim 1, **characterized in that** the applicator tampon (6, 7, 8, 9) is ejected from the assembly channel (2) by means of compressed air that enters the assembly channel (2) through the distal opening of the second section (22).

3. The method as claimed in claim 1 or 2, **characterized in that** the applicator tampon (6, 7, 8, 9) is ejected by means of compressed air that enters the assembly channel (2) through the side channel (3).

## Revendications

1. Procédé de fabrication de tampons applicateurs en utilisant un canal de montage de forme tubulaire (2) avec une première portion (21) ayant un premier diamètre et une deuxième portion (22) ayant un deuxième diamètre plus petit par rapport au premier, les deux portions étant en butée l'une contre l'autre suivant une orientation axiale et formant ainsi un épaulement (23), l'ouverture distale de la deuxième portion (22) s'élargissant en forme d'entonnoir et un canal latéral (3) débouchant dans la deuxième portion (22) à proximité de l'épaulement (23), et en utilisant un système d'air comprimé qui peut être connecté distalement avec l'ouverture distale de la deuxième portion (22) et avec le canal latéral (3) en pressant un tampon (6) avec des fils de retenue de tampon (7) et un petit tube intérieur d'applicateur (9) dans un petit tube extérieur d'applicateur (8) au moyen d'un poinçon de pression (5), **caractérisé en ce que** le petit tube extérieur d'applicateur (8) est positionné dans la première portion (21) du canal de montage (2) de telle sorte que l'ouverture du petit tube extérieur d'applicateur (8) s'applique contre l'épaulement (23), le tampon (6) est enfoncé dans le petit tube extérieur d'applicateur (8) à travers la deuxième portion (22) du canal de montage (2) de telle sorte que le fil de retenue de tampon (7) reste dans la deuxième portion (22) du canal de montage (2), de l'air comprimé est soufflé à travers l'ouverture distale de la deuxième portion (22) dans le canal de montage (2), de telle sorte que le fil de retenue de tampon (7) soit étendu dans le canal latéral (3), le petit tube intérieur d'applicateur (9) est positionné dans la deuxième portion (22) du canal de montage (2) de telle sorte que l'embouchure du canal latéral (3) reste libre, de l'air comprimé est soufflé à travers le canal latéral (3) dans le canal de montage (2) de telle sorte que le fil de retenue de tampon (7) soit étendu dans le petit tube intérieur d'applicateur (9), le petit tube intérieur d'applicateur (9) est poussé dans le petit tube extérieur d'applicateur (8) jusqu'à ce qu'il atteigne sa position finale et ensuite le tampon applicateur (6, 7, 8, 9) est éjecté du canal de montage (2).

2. Procédé selon la revendication 1, **caractérisé en ce que** l'éjection du tampon applicateur (6, 7, 8, 9) hors du canal de montage (2) s'effectue au moyen d'air comprimé qui entre à travers l'ouverture distale de la deuxième portion (22) dans le canal de montage (2).

3. Procédé selon la revendication 1 ou 2, **caractérisé en ce que** l'éjection du tampon applicateur (6, 7, 8, 9) s'effectue au moyen d'air comprimé qui entre à travers le canal latéral (3) dans le canal de montage (2).
